# EUROPEAN PATENT APPLICATION

(11) **EP 3 232 379 A1**
(43) Date of publication of application: **18.10.2017**
(21) Application number: 17163163.3
(22) Date of filing: 27.03.2017
(51) Int. Cl.: G06Q 10/00, G06Q 30/00

(54) **SMART REFRIGERATOR**

(30) Priority: 11.04.2016 TW 105205018 U
(71) Applicant: Prob Jet Tech., Taipei City 114 (TW)
(72) Inventor: Huang, Hsin-Wei, Taipei City 106 (TW); Chou, Chin-Lieh, Taipei City 114 (TW)
(74) Representative: Becker Kurig Straus

(57) **Abstract**

The present disclosure illustrates a smart refrigerator including a user interface, a recipe management module, a storage management module and an order management module. The user interface is configured to input an ordering instruction. The recipe management module stores much recipe information recording information of ingredients required. The user can select at least one of the recipe information. The storage management module is configured to input and store the ingredient information. The order management module is configured to receive and store order information. The recipe management module transmits the selected recipe information to the storage management module, and the storage management module compares the received recipe information and the information of ingredient stored in the refrigerator, so as to generate the order information. When the ordering instruction is inputted into the user interface, the order management module transmits the order information to a cloud server. The smart refrigerator of the present disclosure has advantage of providing the user to more conveniently manage the ingredients stored in the refrigerator, and further providing functions of analyzing recipe and ordering ingredient online.

## Description

### BACKGROUND OF THE INVENTION

### 1. Field of the Invention

The present disclosure relates to a smart refrigerator, more particularly to a smart refrigerator capable of managing ingredient storages, analyzing recipe and ordering ingredient through network.

### 2. Description of the Related Art

A refrigerator, which is one of common and popular appliances in every house or every business place, is used to store various foods or drinks under low temperature or make ice cubes. However, the traditional refrigerator only has function of storing foods.

After placing foods in the refrigerator, the consumer may forget which food is stored in the refrigerator, so that the foods stored are usually neglected, which results in purchase of similar foods or storage of excessive foods. Under the condition of being negligent of management, the food is usually stored in the refrigerator beyond its expiration date, which causes food waste. Furthermore, it spends a lot of time for the consumer to clean the refrigerator, and the refrigerator is hard to maintain its internal temperature if its door is opened for a long term. On the one hand, cleaning refrigerator increases the power consumption of the refrigerator; on the other hand, the freshness of food may be affected during the process of cleaning refrigerator.

With progress of technology, the refrigerator only having food storage function without food analysis or health management function, cannot satisfy the health-conscious consumer.

Therefore, what is need is to develop the refrigerator capable of conveniently managing the food stored inside.

### SUMMARY OF THE INVENTION

In order to solve the above-mentioned problem of the traditional refrigerator, the present disclosure is to provide a smart refrigerator capable of providing the user to more conveniently manage the ingredients stored in the refrigerator, and further providing functions of analyzing recipe and ordering ingredient online.

According to one exemplary embodiment of the present disclosure, a smart refrigerator includes a user interface, a recipe management module, a storage management module and an order management module. The user interface is configured to provide a user to input an ordering instruction. The recipe management module is configured to store multiple recipes which record information of multiple ingredients required for the recipes respectively, and provide the user to select at least one recipe. The storage management module is configured to provide the user to input and store information of the ingredient. The order management module is configured to receive and store information of at least one order. The recipe management module transmits the selected recipe to the storage management module, and the storage management module receives information of the selected recipe, and then compares and analyzes the information of the selected recipe with the stored ingredient information, so as to obtain information of lacking ingredient for the selected recipe and generate the order information. When the ordering instruction is inputted to the user interface, the order management module transmits the order information to a cloud server through internet.

Preferably, the smart refrigerator further includes a health management module configured to provide the user to input user information, and the health management module analyzes the information of the selected recipe according to the user information, and the user interface shows an analysis result.

Preferably, the user information includes gender, age, height, weight and BMI value, and the analysis result generated from the health management module comprises calories of cuisine, and recommended exercise type and time.

Preferably, upon receipt of the order information, the cloud server transmits the order information to at least one manufacturer terminal corresponding thereto.

Preferably, the recipe management module downloads the recipe information from the cloud server.

Preferably, the smart refrigerator further includes a wireless network module, and the smart refrigerator is linked with the internet through the wireless network module.

Preferably, the smart refrigerator further includes a wired network module, and the smart refrigerator is linked with the internet through the wired network module.

Preferably, the ingredient information is inputted into the storage management module by a manner of voice recognition or manual operation.

Preferably, the cloud server is further linked to at least one mobile application program, and the mobile application program accesses the ingredient information and the order information through the cloud server, and the mobile application program is configured to input the ordering instruction.

### BRIEF DESCRIPTION OF THE DRAWINGS

The detailed structure, operating principle and effects of the present disclosure will now be described in more details hereinafter with reference to the accompanying drawings that show various embodiments of the present disclosure as follows.
Fig. 1 shows a smart refrigerator of the present disclosure.
Fig. 2 shows a structural block diagram of the smart refrigerator of the present disclosure.
Fig. 3 shows a schematic view of a storage management module of the present disclosure.
Fig. 4 shows a recipe management module of the present disclosure.
Fig. 5 shows a schematic view of an order management module of the present disclosure.
Fig. 6 shows a schematic view of a health management module of the present disclosure.

### DETAILED DESCRIPTION OF THE PREFERRED EMBODIMENTS

Reference will now be made in detail to the exemplary embodiments of the present disclosure, examples of which are illustrated in the accompanying drawings. Therefore, it is to be understood that the foregoing is illustrative of exemplary embodiments and is not to be construed as limited to the specific embodiments disclosed, and that modifications to the disclosed exemplary embodiments, as well as other exemplary embodiments, are intended to be included within the scope of the appended claims. These embodiments are provided so that this disclosure will be thorough and complete, and will fully convey the inventive concept to those skilled in the art. The relative proportions and ratios of elements in the drawings may be exaggerated or diminished in size for the sake of clarity and convenience in the drawings, and such arbitrary proportions are only illustrative and not limiting in any way. The same reference numbers are used in the drawings and the description to refer to the same or like parts.

It will be understood that, although the terms 'first', 'second', 'third', etc., may be used herein to describe various elements, these elements should not be limited by these terms. The terms are used only for the purpose of distinguishing one component from another component. Thus, a first element discussed below could be termed a second element without departing from the teachings of embodiments. As used herein, the term "or" includes any and all combinations of one or more of the associated listed items.

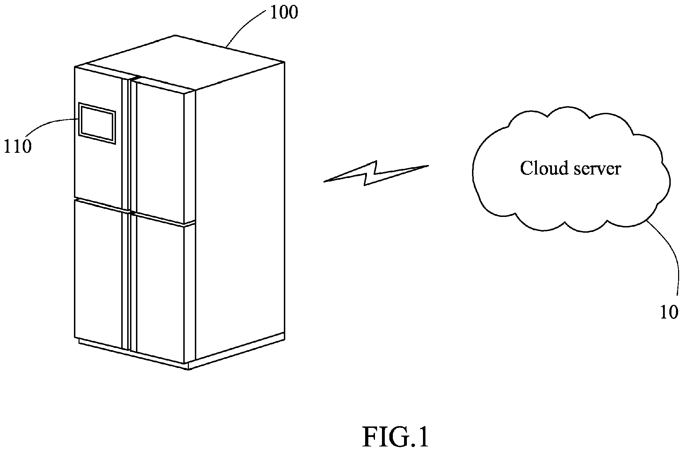

Please refer to Fig. 1 which shows a smart refrigerator 100 of the present invention. The smart refrigerator 100 has a user interface 110 disposed at an outer surface thereof, and the user can control the smart refrigerator 100 through the user interface 110 to operate smart features of the smart refrigerator 100. The smart refrigerator 100 can be linked to a cloud server 10 through internet, so as to provide different smart features.

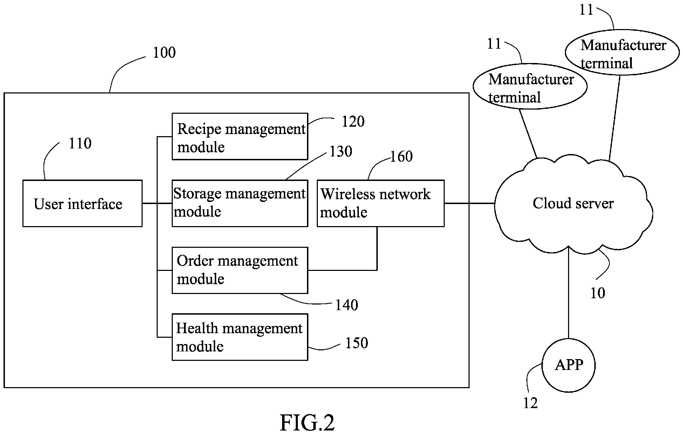

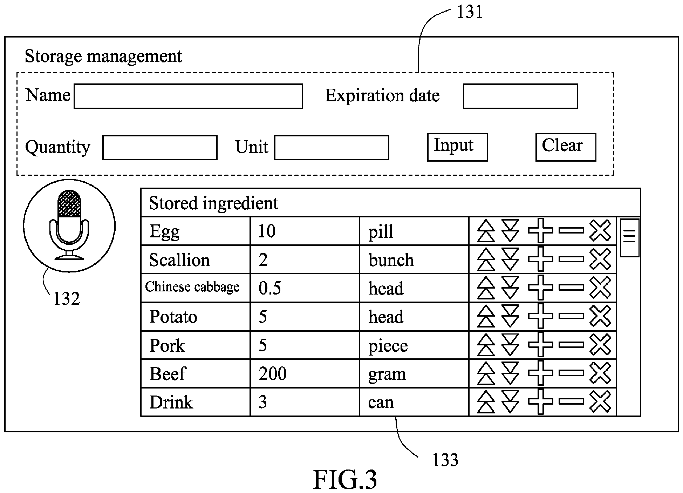

Please refer to Fig. 2 which shows a structural block diagram of the smart refrigerator 100. The smart refrigerator 100 includes the user interface 110, a recipe management module 120, a storage management module 130, an order management module 140, a health management module 150 and a wireless network module 160. The smart refrigerator 100 is linked with the cloud server 10 through internet, and the cloud server 10 is in communication connection with a plurality of manufacturer terminals 11 and mobile application programs 12 respectively. In this embodiment, the smart refrigerator 100 is linked with the internet through the wireless network module 160, such as Wi-Fi wireless network, but the present disclosure is not limited thereto. For example, in some embodiments, the smart refrigerator 100 can be linked with the internet through wired network. The user interface 110 is configured to operate functions of the recipe management module 120, the storage management module 130, the order management module 140 and the health management module 150. In some embodiments, the user interface 110 can be configured to control parameters or functions of the smart refrigerator 100, such as functions of controlling temperature, sterilizing or making ice. The way of using the recipe management module 120, the storage management module 130, the order management module 140 and the health management module 150 will be described in detail below. It should be noted that the operation interfaces shown in Figs. 3-6 of the present disclosure are just taken as examples for illustration, and a person skilled in the art can change the design of the operation interface upon practical demand.

The storage management module 130 is configured to provide the user to input and store the ingredient information, that is, user can input the ingredient information through the storage management module 130 after placing the ingredient in the smart refrigerator 100. Therefore, the ingredient information stored in the storage management module 130 indicates the ingredients stored in the smart refrigerator 100 currently. Please refer to Fig. 3 which shows a schematic view of the storage management module. The user can input the ingredient information through an input area 131, and the ingredient information includes a name, a number, a unit, an expiration date of the ingredient. In this embodiment, the storage management module 130 further includes a voice button 132, and the user can press the voice button 132 for inputting the ingredient information by voice. In other words, in this embodiment the ingredient information can be inputted through a manner of voice recognition or manual operation. The input ingredient information is shown in the ingredient list 133 below. The ingredient list 133 shows the ingredient information stored currently and the user can edit the ingredient information shown in the ingredient list 133 by instructions, such as move-up, move-down, increase/decrease the number of the ingredient or delete instruction.

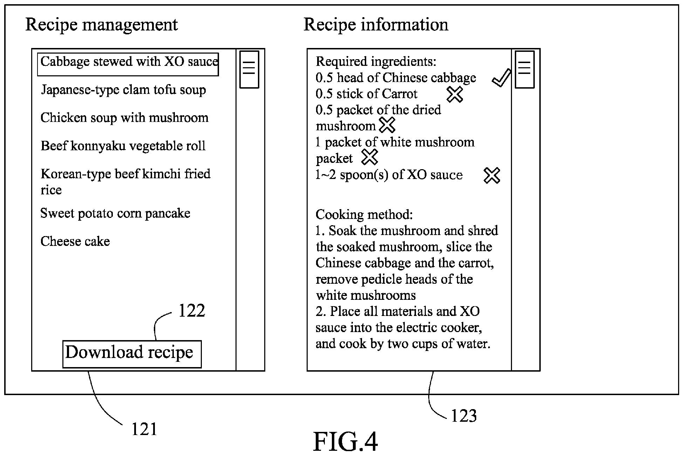

The recipe management module 120 stores the information of many recipes, and the information of each recipe records information of ingredient required for the recipe. The user can select information of at least one recipe from the recipe management module 120. Please refer to Fig. 4 which shows the recipe management module 120. The user can select different recipe information from the recipe list 121 shown at left side, and content of the selected recipe information, including the ingredient and cooking method for the selected recipe, is shown in the information field 123 at right side. In this embodiment, the recipe list 121 further includes a download button 122 shown at a lowermost part thereof, and the user can click the download button 122 to trigger the recipe management module 120 to link the cloud server 10 for downloading more information associated with new recipes.

The selected recipe information is transmitted to the storage management module 130. The storage management module 130 compares the received recipe information with the information of the ingredients stored in the refrigerator, so as to analyze the information of the lacking ingredient for the selected recipe and generate order information corresponding thereto. In other words, the storage management module 130 analyzes whether the ingredients currently stored in the smart refrigerator 100 are sufficient for the selected recipe, and shows the lack of which ingredient for reminding the user to buy. The generated order information includes the ingredient which is lack for the selected recipe. Through the smart refrigerator 100, the user can order on internet to buy the ingredient required.

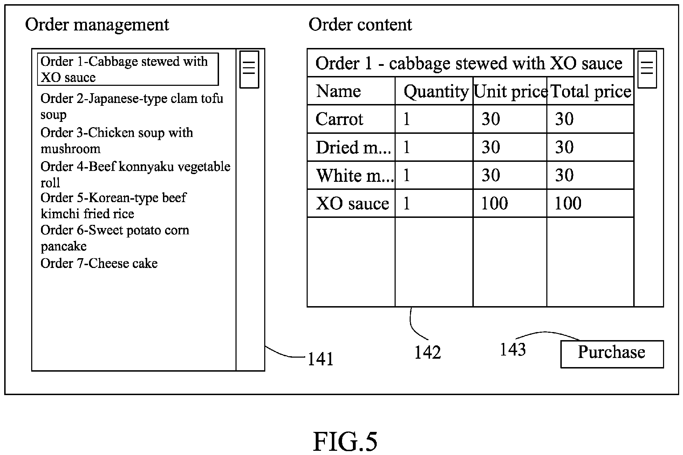

The order information generated by the storage management module 130 is transmitted to the order management module 140, and the order management module 140 receives and stores the order information. Please refer to Fig. 5 which shows a schematic view of the order management module. The user can view the generated order information in the order management module 140, and select one of the order information in the order list 141 shown in the left side. The content of the selected order information is shown at an order details field 142 at the right side. The content of the order information includes names, numbers, units, prices, and a total price of the ingredients. In some embodiments, the user can edit the order information in the order details field 142, for example, edition of the ingredient, the number of the ingredient, the brand or the supplier for the ingredient. After edition, the user can click a purchase button 143 on the user interface 110 to input the ordering instruction, and the order management module 140 then transmits the order information to the cloud server 10 through internet.

Upon receipt of order information, the cloud server 10 respectively transmits the order information to the manufacturer terminals 11 corresponding to the content of the order information, so as to notify the manufacturer terminal 11 to deliver the ingredient which is listed in the order, to the user. In some embodiments, the cloud server 10 is further in communication connection with financial institutions or third-party payment institution. In other words, the user can register payment solution of credit card or third-party payment institutions in the smart refrigerator 100, so that the user can directly pay after using the order management module 140 to order, and it effectively improves the convenience in purchase of ingredients.

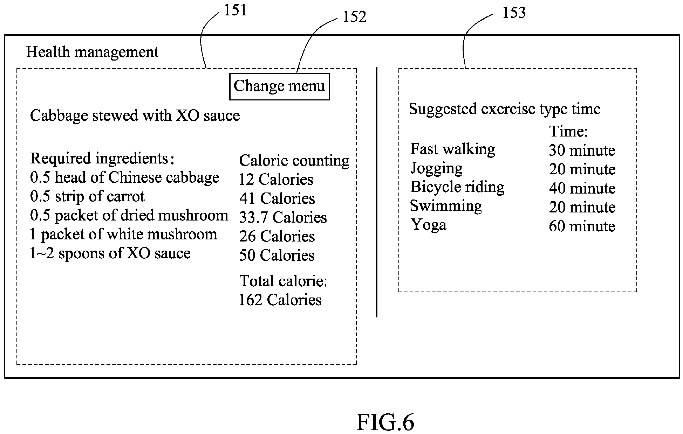

The health management module 150 is configured to provide the user to input user information which includes gender, age, height, weight and BMI value. The health management module 150 is configured to analyze the recipe information according to the user information, and display an analysis result on the user interface 110. Please refer to Fig. 6 which shows a schematic view of the health management module 150. As shown in Fig. 6, the recipe information is loaded into the health management module 150, and the health management module 150 performs determination for the ingredients described in the recipe information, and then calculates the calories of the cuisine according to the ingredients described in the recipe information, and lists and displays calories of each ingredient and total calories of all ingredients in the analysis field 151 at the left side. In addition, the analysis field 151 further includes a menu replacement button 152, so that the user can change the analysis target through the menu replacement button 152.

Next, the health management module 150 evaluates the calorie consumption according to user information, and recommends and displays exercise type and time in the recommendation field 153 at right side. Therefore, the user can be reminded about the calories of the recipe and which exercise type and time suitable for consuming the calorie intake. In other words, the smart refrigerator 100 further includes an ingredient calorie database and an exercise calorie consumption database. The ingredient calorie database records calories of various ingredients, and the exercise calorie consumption database records the calorie consumptions of various exercises for the user with different gender, weight and exercise time. The health management module 150 performs analysis according to the ingredient calorie database and the exercise calorie consumption database, so as to calculate the calorie of the cuisine and recommend suitable exercise type and exercise time for the user.

Please refer back to Fig. 2. The cloud server 10 is further in communication connection with a mobile application program (APP) 12. The mobile application program 12 can be linked with the cloud server 10 and the smart refrigerator 100 to read the ingredient information and order information, and the ordering instruction can also be inputted into the mobile application program 12. In other words, the user can install the mobile application program 12 in a mobile device such as a smart phone or a tablet computer first, and then check the status of the smart refrigerator 100 through the mobile application program 12. For example, the user can check the ingredient stored in the smart refrigerator 100 through the mobile application program 12 while buying ingredients outdoors, so as to prevent from buying excessive or similar ingredients. Or, the user can check the recipe information through the mobile application program 12 for making sure which ingredient to buy.

The above-mentioned descriptions represent merely the exemplary embodiment of the present disclosure, without any intention to limit the scope of the present disclosure thereto. Various equivalent changes, alternations or modifications based on the claims of present disclosure are all consequently viewed as being embraced by the scope of the present disclosure.

## Claims

1. A smart refrigerator, comprising:
a user interface configured to provide a user to input an ordering instruction;
a recipe management module configured to store information of multiple recipes recording information of multiple required ingredients respectively, and provide the user to select the information of at least one recipe;
a storage management module configured to provide the user to input and store information of the ingredient; and
an order management module configured to receive and store information of at least one order;
wherein, the recipe management module transmits the information of the selected recipe to the storage management module, and the storage management module receives the information of the selected recipe, and then compare and analyze the selected recipe with the stored information of ingredients, so as to obtain information of lacking ingredient for the selected recipe and generate the order information;
wherein when the ordering instruction is inputted to the user interface, the order management module transmits the order information to a cloud server through internet.

2. The smart refrigerator according to claim 1, further comprising a health management module configured to provide the user to input user information, wherein the health management module analyzes the selected recipe according to the user information, and the user interface shows an analysis result.

3. The smart refrigerator according to claim 1, wherein the user information comprises gender, age, height, weight and BMI value, and the analysis result generated from the health management module comprises calories of cuisine, and recommended exercise type and time.

4. The smart refrigerator according to claim 1, wherein upon receipt of the order information, the cloud server transmits the order information to at least one manufacturer terminal corresponding thereto.

5. The smart refrigerator according to claim 1, wherein the recipe management module downloads the recipe information from the cloud server.

6. The smart refrigerator according to claim 1, further comprising a wireless network module, wherein the smart refrigerator is linked with the internet through the wireless network module.

7. The smart refrigerator according to claim 1, further comprising a wired network module, wherein the smart refrigerator is linked with the internet through the wired network module.

8. The smart refrigerator according to claim 1, wherein the ingredient information is inputted into the storage management module by a manner of voice recognition or manual operation.

9. The smart refrigerator according to claim 1, wherein the cloud server is further linked to at least one mobile application program, and the mobile application program accesses the information of ingredients and the order information through the cloud server, and the mobile application program is configured to input the ordering instruction.
